(19) **Europäisches Patentamt** / **European Patent Office** / **Office européen des brevets**

(11) **EP 2 124 975 B1**

(12) **EUROPEAN PATENT SPECIFICATION**

(45) Date of publication and mention
of the grant of the patent:
**06.10.2010 Bulletin 2010/40**

(21) Application number: **08701835.4**

(22) Date of filing: **17.01.2008**

(51) Int Cl.:
***A61K 35/64*** *(2006.01)*     ***A61P 31/04*** *(2006.01)*

(86) International application number:
**PCT/GB2008/000157**

(87) International publication number:
**WO 2008/087416 (24.07.2008 Gazette 2008/30)**

(54) **ANTIMICROBIAL COMPOSITION FOR CONTROLLING CONTAMINATION AND INFECTION**

ANTIMIKROBIELLE ZUSAMMENSETZUNG ZUR KONTAMINIERUNGS- UND
INFEKTIONSKONTROLLE

COMPOSITION ANTIMICROBIENNE POUR LE CONTRÔLE DE CONTAMINATION ET
D'INFECTION

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **18.01.2007  GB 0700946**

(43) Date of publication of application:
**02.12.2009  Bulletin 2009/49**

(73) Proprietor: **UWS Ventures Limited
Swansea
SA2 8PP (GB)**

(72) Inventors:
• **BEXFIELD, Alyson
  Newcastle Upon Tyne NE15 9BN (GB)**
• **BOND, Alison, Elisabeth
  Swansea SA7 9BU (GB)**

• **DUDLEY, Edward
  Swansea SA4 9ZA (GB)**
• **NEWTON, Russel, Peter
  Swansea (GB)**
• **NIGAM, Yamni
  Swansea SA2 0LS (GB)**
• **RATCLIFFE, Norman, Arthur
  Swansea SA2 8PP (GB)**

(74) Representative: **Ratcliffe, Susan Margaret et al
Department of Research and Innovation
Faraday Building 7th Floor
Swansea University
Singleton Park
Swansea SA2 8PP (GB)**

(56) References cited:
**WO-A-01/31033     WO-A-03/075654**

**Description**

The present invention relates to an antimicrobial composition for use in methods of controlling contamination and infection using said composition.

Antimicrobial compositions are well known and can be used to treat a range of organisms including bacteria, fungi, yeast and protozoa. Antimicrobials can be obtained from a range of sources for example from plants, from extractions isolated from microbial cultures or from secretions from invertebrates such as insects. It is known that an extract from the larvae of the green-bottle fly (*Lucilia sericata*) has antimicrobial properties.

Also, many different substances have been proposed to be used in the healing of wounds. These substances include collagenase, streptodornase and streptokinase which are obtained from bacterial sources. Other substances include Bromelain, obtained from pineapples, Krill enzymes which are obtained from crustaceae and plasmin and trypsin, which are typically obtained from mammalian sources such as cattle.

A problem with many antibiotics or healing agents is that they are often only partially effective. In particular, in the case of antibiotics, as a result of widespread and indiscriminate use, microbes have become *resistant* to the microbial effects of such antibiotic compounds. Particular antibiotic *resistant* "*superbugs*" include methicillin-resistant *Staphylococcus aureus* (MRSA) and vancomycin-resistant *Enterococcus* (VRE). In the USA, vancomycin-resistant strains of *Staphylococcus* aureus have also been found, which illustrates that bacteria are becoming resistant to a greater range of antibiotics. If a person is infected with such resistant organisms healing can be very prolonged and costly. Also, as the bacteria have built up a resistance to most of the commonly used antibiotics then the infection may never be fully controlled, which may result in a patient having an amputation of the infected area. The cost of these more intense treatments increases the financial burden on health care and, in particular, State health care provision. It is estimated that the cost of treating wound infections to the UK National Health Service is at least £1 billion annually and the emergence of antibiotic resistant microorganisms contributes to these costs.

It is known that the treatment of wounds with larvae from green-bottle flies can provide an effective and simple way of *cleansing* infected and necrotic wounds. The application of sterile larvae from *Lucilia sericata* to an infected wound results in the removal of necrotic and sloughy tissue, disinfection and enhancement of the healing process. The process of applying sterile fly larvae to wounds is generally known as maggot therapy.

Although it is recognised that there is a degree of antimicrobial action involved in maggot therapy, there has been much dispute as to how this occurs. It is thought that the process may be by a mechanical action for example mechanical irrigation of the wound by the physical digestion of tissue by the larvae, together with ingestion of bacteria by the larvae. Alternative theories are that excretions/secretions (ES) produced by the larvae may contain factors that may disinfect the wound.

A problem with maggot therapy is that the application of live maggots to the body is not acceptable to some patients and clinicians. They have a fear that the maggots may burrow under the skin and some imagine that they can feel the maggots moving in situ or are afraid that they could escape from a wound dressing. Although none of these events actually occur, the perception of them occurring is a real deterrent to some people accepting maggot therapy.

Investigations have been made to establish how maggot therapy leads to antimicrobial activity but although some studies recognise that the extracorporeal excretions/secretions of maggots may contain an antibiotic, there is no indication of what this is or whether it could be isolated from the exudates from the maggots.

In WO 03/07654, which covers work done by The University of Nottingham in the UK, it has been shown that the secretions/excretions of *Lucilia sericata* have N-acyl homoserine lactone degrading activity, which may be due to a serine proteinase or glycosidase. However, the compositions discussed are made up of the complete excretion/secretion from the maggots, there is no purified component that has been isolated, which can be used as an antimicrobial composition in its own right. Further, WO 03/07654 states that excretions/secretions produced from *L.sericata* grown under sterile conditions have no antibiotic effect on the growth of either Gram+ve or Gram-ve bacteria (*S.aureus* and *Ps.aeruginosa* respectively). The excretions/secretions discussed are used with a conventional antibiotic such as tetracyline because the combined effect of the larval secretions and the antibiotic are greater than when they are used separately.

In DE 10149153 A1, DE 10138303 A1, DE 19901134 A1 it is described that enzymes or enzymatic extracts from whole body homogenates of the larvae or pupae of *Sarcophaga* or *Lucilia* can be applied in the treatment of acute or chronic wounds. However, for the compositions discussed there is no specific purified component that has been isolated, which can be purified and used as an antimicrobial in general. Again, having a complete exudate from maggots may be unacceptable to medical personnel and patients alike.

The present seeks to overcome the problems of the prior art by providing a defined antimicrobial composition having a powerful antimicrobial activity, in particular in relation to antibiotic resistant microbes.

It is to be understood that the term antimicrobial refers to compounds and combinations of compounds that have bacteriostatic as well as *bacteriocidal* properties and which could be administered to a human or an animal to inhibit microbial infection. Although the antimicrobial is particularly applicable to wounds infected with bacteria, it may be used to treat other infections, for example fungal infections or infections where there is a mixture of bacteria and other organisms. Also, the antimicrobial may be administered topically or systemically for the treatment or prevention of infection. The antimicrobial may also be used in other applications such as, but not limited to, the prevention of food spoilage, as a disinfectant, an antiseptic, surface cleaner or preservative.

In short, the current invention is concerned with a particular defined compound that has been isolated from maggots which has the unexpected effect of being an antimicrobial that can be used in a wide range of applications. Examples of these applications, which is not to be considered to be a limited list include use as an antimicrobial in the treatment of wounds, which can be either topically or intravenously administered or to a general antimicrobial that can prevent contamination in a wide range of situations.

According to a first aspect of the present invention there is provided an antimicrobial composition having the empirical formula $C_{10}H_{16}N_6O_9$. This composition has been named by the inventors as Seraticin™.

It is preferred that the antimicrobial composition is an isolate from excretions/secretions of fly larvae although it is envisaged that it may be isolated from other sources such as vertebrates or invertebrates.

Preferably, the antimicrobial composition is obtained from fly larvae of the species *Lucilia sericata*.

The invention also relates to a pharmaceutical compositions, which comprises combining the fly larval composition of the invention into a suitable dosage form with a pharmaceutically suitable and physiologically tolerated carrier and, where appropriate, other suitable active substances, additives or excipients.

The invention also relates to the use of the compositions for producing pharmaceuticals. The pharmaceuticals can be used for the therapy of superficial or deep chronic and acute wounds of any etiology.

The pharmaceuticals of the invention are usually applied topically but they may be applied intracorporally, such as intravenously, or by implants in the body or by a transdermal application or even by way of oral application.

Suitable pharmaceutical compositions for topical use on the skin are preferably in the form of a solution, suspension, dusting powder, liposomal formulations, gel, lotion, paste, spray or aerosol. Carries which can also be used are polyethylene glycols, alcohols and combinations of two or more of these substances. The list can by no means be regarded as restrictive. The fly larval microbial extracts of the invention are present in a concentration of from 0.1% by weight to 100% by weight of the composition, for example 50-90%, or 40-80%, 30-70% or from 1.0% by weight to 60% by weight, depending on the extraction conditions.

Transdermal administration is also possible. Suitable pharmaceutical compositions for transdermal uses may be in the form of individual plasters which are suitable for long-term close contact with the patient's epidermis. Plasters of this type suitably contain the microbial composition of the invention in an optionally buffered aqueous solution, dissolved and/or dispersed in an adhesive or dispersed in a polymer. A suitable active substance concentration is from about 0.01% by weight to 75% by weight, preferably from 1% by weight to 70% by weight.

The antimicrobial of the invention can also be applied to the wound through wound coverings made of gauze, of alginates, of hydrocolloid materials, foams and silicone coverings, which have been coated, impregnated or treated with the composition and are therefore able to deliver the composition into or onto the wound or wound surface.

The composition of the invention may also be delivered as an oral pharmaceutical such as a tablet.

Suitable solid pharmaceutical forms are, for example, granules, powders, solutions, suspensions, emulsions or drops, and the products with protracted release of active substance, in the production of which conventional excipients or carriers are used. Excipients which are frequently used and which may be mentioned are magnesium carbonate, titanium dioxide, lactose, mannitol and other sugars, talc, milk protein, gelatine, starch, cellulose and derivatives thereof, polyethylene glycol and solvents such as, for example, sterile water and monohydric or polyhydric alcohols such as glycerol.

According to a further aspect, the invention relates to a pharmaceutical composition arranged to be administered intravenously.

The carrier composition may be any pharmaceutically acceptable carrier such as a solvent for antibiotics, chelating agents and pH buffering agents that will allow a therapeutic composition to be administered directly to the skin, wound or mucosa or systemically by, for example but not limited to, oral or intravenous administration. The carrier may also allow the use of the antimicrobial for other purposes, for example but not limited to, antiseptics, disinfectants and preservatives. Examples of carriers that can be used, which is not a limiting list, include water, saline, physiological saline, ointments, creams, oil in water emulsions, gels, solvents or combinations of solvents.

The treatment preparation also includes a pH buffering agent. The term pH buffering agent includes, for example, pharmaceutically acceptable organic or inorganic compounds or combinations of compounds that will maintain the pH of the treatment preparation within desired pH limits.

It is envisaged that the treatment preparation further includes a surfactant. Known surfactants, such as detergents, or anionic or ionic surfactant or a mixture thereof may be used. Surfactants are included to alter the surface tension of aqueous solutions containing the antimicrobial composition and therefore provide a treatment preparation that can be easily taken into the body.

It is envisaged that the carrier may include one or more of a pH buffering agent, a tissue growth promoting agent, an anti-inflammatory or a pain killer and a surfactant or any such agents which allow administration via, for example, oral, parenteral or intravenous methods.

The treatment preparation may also include a wound healing promoter such as Vitamin E which can assist in the skin around a wound healing by promoting tissue growth.

In addition the treatment preparation may include, such as, one or more anti-inflammatory agents for example a corticosteroid or agents that can reduce pain, such as morphine based substances. These inclusion are particularly useful if the patient is feeling uncomfortable because the wound damage is deep seated.

In addition the antimicrobial composition may be incorporated in a formulation to treat food to reduce the risk of microbial contamination.

Further the antimicrobial may be incorporated in a disinfectant to be used as a general surface cleaner, for example, hard surfaces in bathrooms, kitchens and on floors.

The Antimicrobial composition may also be used as a general antiseptic, which can be used in a range of environments, not only in the house, but in hospitals, workplaces, in areas where other sterile environments are required.

The Antimicrobial may be incorporated in for example antiseptic wipes or in misters or it can be used as a liquid or gel in the form of a surface cleaner.

Further, the composition claimed when used as a medicament may be provided in the form of a salt, an ester or pro drug derivative.

There is also provided a method of treating a wound infection, said method comprising contacting a wound with a therapeutic amount of an antimicrobial composition of the empirical formula $C_{10}H_{16}N_6O_9$. Furthermore, infection may be treated by, but not limited to, oral, intravenous, or parenteral administration of the antimicrobial composition.

Preferably, the antimicrobial composition is an isolate from the secretions/excretions of fly larvae.

It is envisaged that preferably the species of fly larvae is *Lucilia sericata*.

Preferably, the Antimicrobial composition is included in a pharmaceutically acceptable carrier.

It is envisaged that the carrier may include one or more of a pH buffering agent, a tissue growth promoting agent and a surfactant.

Treatment is preferably oral, or failing that, parenteral.

According to yet further aspect of the invention, there is provided a wound dressing including an antimicrobial composition having the empirical formula of $C_{10}H_{16}N_6O_9$.

Preferably the wound dressing includes a treatment preparation including an antimicrobial composition of the empirical formula $C_{10}H_{16}N_6O_9$ and a carrier composition for the antimicrobial composition.

It is envisaged that the carrier may include one or more of a pH buffering agent, a tissue growth promoting agent, an anti-inflammatory or a pain killer and a surfactant.

Although separate aspects or embodiments of the invention have been described, the invention is intended to also cover combinations of those aspects or embodiments.

The following examples are provided to illustrate preferred embodiments and uses of the present invention but should not be construed to limit the scope of the invention.

Example 1

METHODS AND MATERIALS

Fly Larvae

Sterile third instar larvae of *Lucilia sericata* were supplied by the Zoobiotic Limited, Bridgend, UK.

Microorganisms

A range of Gram-positive and Gram-negative bacteria and fungi, including but not limited to Methicillin-resistant *Staphylococcus aureus* (MRSA) *Staphylococcus Sp., Bacillus sp., Escherichia coli, Pseudomonas sp.,* Proteus *sp., Enterococcus sp., Serratia sp., Candida* sp. and Enterobacter sp were purchased from the National Collections of Industrial and Marine Bacteria (NCIMB) Aberdeen, UK or provided by the Pathology Laboratory of the Princess of Wales Hospital, Bridgend, UK.

Collection of larval excretions/secretions

Native excretions/secretions (nES) were collected by incubating third-instar larvae in a small quantity (200 μl g$^{-1}$ of larvae) of sterile Milli-Q ultra pure water (Millipore UK Ltd, Hertfordshire, UK) for 1 h at 30°C in darkness. The sterile liquid was siphoned off from the containers and centrifuged at 10,000 xg for 5 minutes to remove particulate material, after which the supernatant was retained at -20 to -80°C for testing. Although third-instar larvae were used, it is possible to use first and second instar-larvae but third instar-larvae yielded increased antimicrobial activity and sample volume.

Purification of the supernatant

A pooled volume of nES was fractionated in an Amicon stirred ultrafiltration cell (Millipore UK Ltd) that was pressurised with $N_2$ gas. The Amicon filters generated fractions from the excretions/secretions of different molecular weights - (Bexfield, A., et al., Microbes and Infection 6, 1297-1304, - 2004).

Separation of Fractions

Fractions of maggot excretion/secretion filtrate having molecular weights of <500 Da were concentrated 10 times using an Eppendorf Speedvac vacuum centrifuge at 25-35°C prior to any HPLC separation.

Initially, 100μl of the concentrated <500Da filtrate was injected onto a C30 HPLC column and guard column from YMC Europe GmbH (Germany) using a Vision Workstation (Applied Biosciences, U.K).

The mobile phase consisted of an aqueous mobile phase A (98% Milli - Q water, 2% methanol) and an organic mobile phase B (methanol). A gradient was applied to run from 100% A to 100% B over 30 minutes and remained at 100% B for 30 minutes and then the column was re-equilibrated in 100% A for 40 minutes. The eluent was then passed to the built-in fraction collector and the subsequent collected fractions were then 0.2-0.4μm filtered to sterilise and assayed for antibacterial activity using the Turbidometric assay (TB) now described.

Antimicrobial Assays

Bacteria were grown for 17 hours in 20ml of tryptic soy broth (TSB). The bacteria were grown at 30°C with the culture being oscillated at a rate of 90 oscillations per minute. Following growth, the bacteria were washed in phosphate buffered saline, pH 7.4, and adjusted to a concentration of $2x10^5$ bacteria per ml in fresh TSB.

To measure antibacterial activity, a turbidometric (TB) assay was carried out. One hundred and fifty microlitres of each HPLC fraction was mixed with 16.7 μl of 10% peptone water, pH 8.5, and 50μl were then incubated with 10μl of bacterial suspension in triplicate in the wells of a sterile, flat-bottom, 96-well microtitre plate. The samples were incubated at 37°C for 24 hours and the optical density at 550nm ($OD_{550}$) measured at hourly intervals from time zero. Controls replaced HPLC fractions with sterile ultrapure water.

All data points were measured against time zero. The survival index (SI) was then calculated as follows to express the growth of bacteria in the HPLC fractions as a percentage of growth in control samples.

$$SI = \frac{OD_{550} \text{ at mid-log of control bacterial growth}}{OD_{550} \text{ of test samples at corresponding time point}} \times 100$$

Detection of Antimicrobial Activity

Antimicrobial activity was collected between 40.5 - 41.0 minutes, and further separation was conducted by injecting 100μL onto a BioBasic SEC - 60 HPLC column from Thermo Electron Corporation (U.K).

For this further separation of components, the mobile phase consisted of 100% Milli - Q water and the flow rate altered step-wise from 0.25mL/min to 1.5mL/min over 60 minutes. Fractions were collected using the built-in fraction collector and assayed for anti-bacterial activity.

Antibacterial activity against MRSA, *S.aureus* and *E. coli* (representative Gram +ve and Gram -ve bacteria) was seen in fractions collected between 44-48 minutes and these fractions were further examined by mass spectrometry.

Accurate mass determination was performed on a Q-ToF Ultima mass spectrometer (Micromass, Manchester, U.K) using a lockspray source. The fractions were introduced to the Q-ToF MS using a LC Packings Ultimate HPLC system (Dionex, Amsterdam, Netherlands). A FAMOS microautosampler equipped with a 1μL loop and a full loop injection program was used for injections. From the FAMOS loop the samples were passed directly into the MS through fused silica tubing with the Chromeleon software suite used for instrument control and for the triggering of MS data acquisition.

Sample introduction was delivered with a mobile phase of 50% methanol in Milli - Q water and 0.1% formic acid. The reference standard used was 2pmol/μL cytidine - 5- monophosphate disodium salt.

The Q-ToF MS was operated in positive ionisation mode with a spray voltage of 3kV, source temperature of 80°C, cone voltage of 35V, MCP voltage set to 2300V and ToF voltage of 9.10kV. The collision energy was set to 4 for full scan spectra experiments and the automated data collection was controlled through the MassLynx program.

The isolated extract from the collection between 44-48 minutes was analysed and the empirical formula so obtained was $C_{10}H_{16}N_6O_9$.

Aspects of the invention are now described with reference to the figures which are provided by way of example only. The figures show that the antimicrobial composition of the invention has the potential to act against a wide range of microbes.

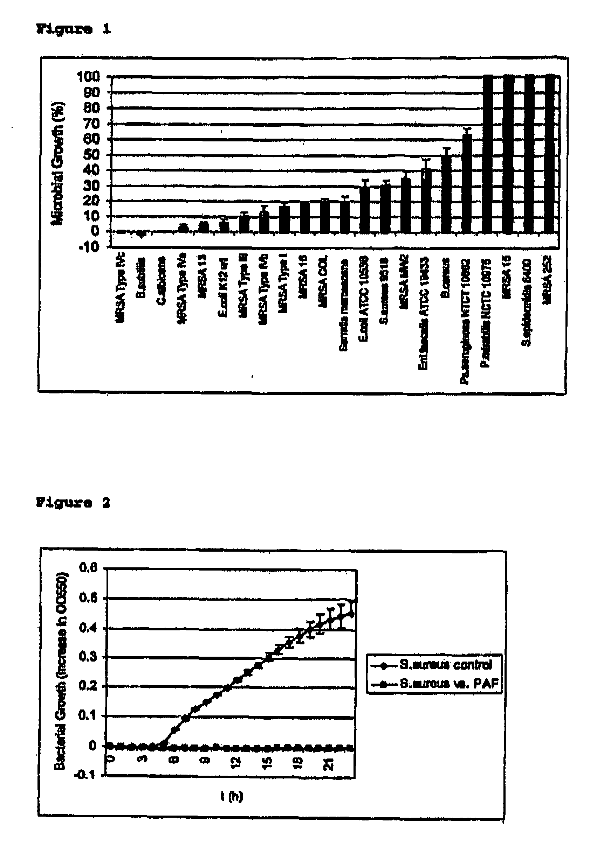

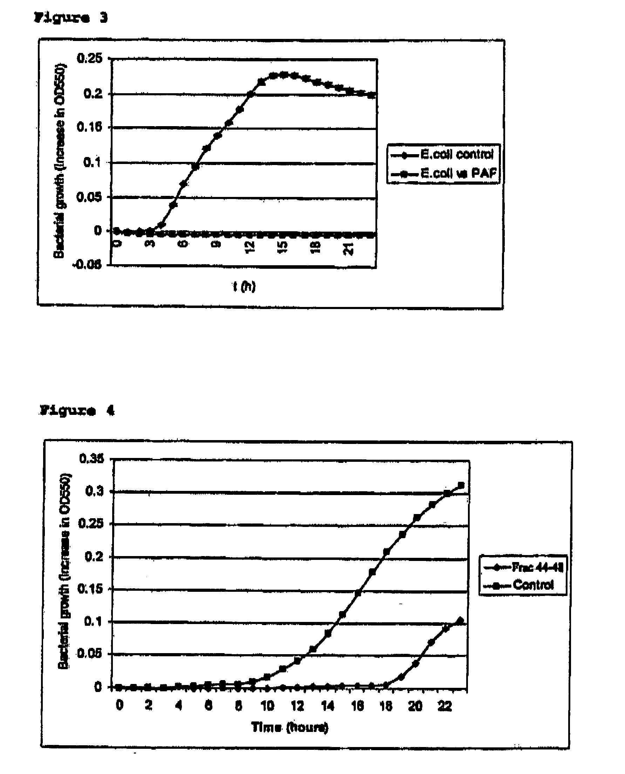

Figure 1 shows antimicrobial activity of <500 Da ultrafiltration fraction (containing seraticin) against a range of Gram+ve (stripe) and Gram-ve (black) bacteria and fungi (Candida albicans). Proteus mirabills, MRSA 15, Staphylococcus epidermidis 8400 and MRSA 252 are all resistant to the antibacterial effects of the <500 Da fraction, however, this may be a concentration issue as further testing of MRSA 252 using purified seraticin (fraction 44-48, figure 4) demonstrated sensitivity to the compound. Bacterial growth is shown as a percentage of control growth.

Figure 2 shows the antibacterial activity of seraticin- containing fractions (SC) from size exclusion chromatography (SEC) and C30 separations against S. aureus.

Figure 3 shows the antibacterial activity of seraticin-containing fractions (SCF) from exclusion chromatography (SEC) and C30 separations against E. coli.

Figure 4 shows the antibacterial activity of 44-48 min fraction against MRSA 252 and this shows clearly that the specific isolate of the empirical formula claimed has an improved antibacterial activity.

The invention covers not only individual embodiments as discussed but combinations of embodiments as well. It is to be understood that modifications and variations of the present invention will become apparent to those skilled in the art and it is intended that all such modifications will be included within the scope of the present invention as defined by the claims. Further, the composition claimed when used as a medicament may be provided in the form of a salt, an ester or pro drug derivative.

## Claims

1. An antimicrobial composition having the empirical formula of $C_{10}H_{16}N_6O_9$.

2. An antimicrobial composition according to claim 1 which is an isolate from invertebrates.

3. An antimicrobial composition according to claim 1 or claim 3 - which is an isolate from the excretions/secretions of fly larvae.

4. An antimicrobial composition according to claim 3, wherein the fly larvae are of the species *Lucilia sericata*.

5. A treatment preparation including an antimicrobial composition according to any preceding claim.

6. A treatment preparation according to claim 5, including carrier composition for the antimicrobial composition.

7. A treatment preparation according to claim 6, wherein the carrier is selected from one or more of the following: water, saline, physiological saline, ointments, creams, oil in water emulsions, gels, solvents or combinations of solvents.

8. A treatment preparation according to claim 7, further including one or more of the following: a chelating agent, a pH buffering agent or a surfactant.

9. A treatment preparation according to claim 7 or claim 8 including one or more wound healing promoters.

**10.** A treatment preparation according to claim 9, wherein the wound healing promoter is Vitamin E.

**11.** A treatment preparation according to any of claims 5 to 10, including one or more anti-inflammatory agents or agents that can reduce pain.

**12.** A wound dressing including an antimicrobial composition having the empirical formula of $C_{10}H_{16}N_6O_9$.

**13.** A wound dressing according to claim 12, wherein the antimicrobial composition is an isolate from the secretions/ excretions of fly larvae.

**14.** A wound dressing according to claim 12, wherein the fly larvae is *Lucilia*.

**15.** A wound dressing according to any of claims 11 to 13, wherein the antimicrobial composition is included in a carrier composition for the antimicrobial, composition.

**16.** A wound dressing according to claim 14, wherein carrier includes one or more of a pH buffering agent, a tissue growth promoting agent, an anti-inflammatory or a pain killer and a surfactant.

**17.** An antiseptic composition including an antimicrobial composition of the formula $C_{10}H_{16}N_6O_9$.

**18.** A disinfectant composition including an Antimicrobial composition of the formula $C_{10}H_{16}N_6O_9$.

**19.** A surface cleaner including an antimicrobial composition of the formula $C_{10}H_{16}N_6O_9$.

**Patentansprüche**

**1.** Antimikrobielle Zusammensetzung mit der empirischen Formel von $C_{10}H_{16}N_6O_9$.

**2.** Antimikrobielle Zusammensetzung nach Anspruch 1, die ein Isolat aus Wirbellosen ist.

**3.** Antimikrobielle Zusammensetzung nach Anspruch 1 oder Anspruch 3, die ein Isolat aus den Exkreten/Sekreten von Fliegenlarven ist.

**4.** Antimikrobielle Zusammensetzung nach Anspruch 3, worin die Fliegenlarven die Spezies *Lucilia sericata* sind.

**5.** Behandlungsmittel, das eine antimikrobielle Zusammensetzung nach einem der vorstehenden Ansprüche einschließt.

**6.** Behandlungsmittel nach Anspruch 5, die eine Trägerzusammensetzung für die antimikrobielle Zusammensetzung einschließt.

**7.** Behandlungsmittel nach Anspruch 6, worin der Träger aus einem oder mehreren der Folgenden ausgewählt ist: Wasser, Kochsalzlösung, physiologischer Kochsalzlösung, Salben, Cremes, Öl-in-Wasser-Emulsionen, Gelen, Lösungsmitteln oder Kombinationen von Lösungsmitteln.

**8.** Behandlungsmittel nach Anspruch 7, das weiter einen oder mehrere der Folgenden einschließt: einen Chelatbildner, einen pH-Puffer oder einen oberflächenaktiven Stoff.

**9.** Behandlungsmittel nach Anspruch 7 oder Anspruch 8, das einen oder mehrere wundheilungsfördernde Mittel einschließt.

**10.** Behandlungsmittel nach Anspruch 9, worin das wundheilungsfördernde Mittel Vitamin E ist.

**11.** Behandlungsmittel nach einem der Ansprüche 5 bis 10, das einen oder mehrere entzündungshemmende Mittel oder Mittel, die Schmerz lindern können, einschließt.

**12.** Wundverband, der eine antimikrobielle Zusammensetzung mit der empirischen Formel von $C_{10}H_{16}N_6O_9$ einschließt.

**EP 2 124 975 B1**

13. Wundverband nach Anspruch 12, worin die antimikrobielle Zusammensetzung ein Isolat aus den Exkreten/Sekreten von Fliegenlarven ist.

14. Wundverband nach Anspruch 12, worin die Fliegenlarven *Lucilia* sind.

15. Wundverband nach einem der Ansprüche 11 bis 13, worin die antimikrobielle Zusammensetzung in einer Trägerzusammensetzung für die antimikrobielle Zusammensetzung eingeschlossen ist.

16. Wundverband nach Anspruch 14, worin der Träger einen oder mehrere von einem pH-Puffer, einem das Gewebewachstum fördernden Mittel, einem entzündungshemmenden Mittel oder einem Schmerzmittel und einem oberflächenaktiven Stoff einschließt.

17. Antiseptische Zusammensetzung, die eine antimikrobielle Zusammensetzung der Formel $C_{10}H_{16}N_6O_9$ einschließt.

18. Desinfizierende Zusammensetzung, die eine antimikrobielle Zusammensetzung der Formel $C_{10}H_{16}N_6O_9$ einschließt.

19. Oberflächenreiniger, der eine antimikrobielle Zusammensetzung der Formel $C_{10}H_{16}N_6O_9$ einschließt.

**Revendications**

1. Composition antimicrobienne ayant la formule empirique de $C_{10}H_{16}N_6O_9$.

2. Composition antimicrobienne selon la revendication 1 qui est un isolat d'invertébrés.

3. Composition antimicrobienne selon la revendication 1 ou selon la revendication 3, qui est un isolat des excrétions/sécrétions des larves de mouche.

4. Composition antimicrobienne selon la revendication 3, dans laquelle les larves de mouche sont de l'espèce *Lucilia sericata.*

5. Préparation de traitement comprenant une composition antimicrobienne selon l'une quelconque des revendications précédentes.

6. Préparation de traitement selon la revendication 5, comprenant une composition de support pour la composition antimicrobienne.

7. Préparation de traitement selon la revendication 6, dans laquelle le support est sélectionné parmi un ou plusieurs éléments parmi les suivants : eau, solution saline, solution saline physiologique, onguents, crèmes, émulsions huile dans l'eau, gels, solvants ou combinaisons de solvants.

8. Préparation de traitement selon la revendication 7, comprenant en outre un ou plusieurs éléments parmi les suivants : un agent chélateur, un tampon pour pH ou un tensioactif.

9. Préparation de traitement selon la revendication 7 ou la revendication 8, comprenant un ou plusieurs promoteurs de cicatrisation de plaie.

10. Préparation de traitement selon la revendication 9, dans laquelle le promoteur de cicatrisation de plaie est la Vitamine E.

11. Préparation de traitement selon l'une quelconque des revendications 5 à 10, comprenant un ou plusieurs agents anti-inflammatoires ou agents qui peuvent amenuiser la douleur.

12. Pansement pour plaie comprenant une composition antimicrobienne ayant la formule empirique de $C_{10}H_{16}N_6O_9$.

13. Pansement pour plaie selon la revendication 12, dans lequel la composition antimicrobienne est un isolat des sécrétions/excrétions de larves de mouche.

**14.** Pansement pour plaie selon la revendication 12, dans lequel les larves de mouche sont des *Lucilia.*

**15.** Pansement pour plaie selon l'une quelconque des revendications 11 à 13, dans lequel la composition antimicrobienne est comprise dans une composition de support pour la composition antimicrobienne.

**16.** Pansement pour plaie selon la revendication 14, dans lequel le support comprend un ou plusieurs éléments parmi un tampon pour pH, un agent de promotion de croissance des tissus, un anti-inflammatoire ou un antidouleur et un tensioactif.

**17.** Composition antiseptique comprenant une composition antimicrobienne de la formule $C_{10}H_{16}N_6O_9$.

**18.** Composition désinfectante comprenant une composition antimicrobienne de la formule $C_{10}H_{16}N_6O_9$.

**19.** Nettoyant de surface comprenant une composition antimicrobienne de la formule $C_{10}H_{16}N_6O_9$.

Figure 1

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0307654 A
- DE 10149153 A1
- DE 10138303 A1
- DE 19901134 A1

**Non-patent literature cited in the description**

- **Bexfield, A. et al.** *Microbes and Infection,* 2004, vol. 6, 1297-1304